# EUROPEAN PATENT SPECIFICATION

(11) **EP 1 709 036 B1**
(45) Date of publication and mention of the grant of the patent: **05.03.2008**
(21) Application number: 05706936.1
(22) Date of filing: 20.01.2005
(51) Int. Cl.: C07D 417/04, C07D 417/14, A61K 31/501, A61K 31/506, A61P 11/00

(54) **THIAZOLE DERIVATIVES AS A2B ANTAGONISTS**
THIAZOLDERIVATE ALS A2B-ANTAGONISTEN
DERIVES DE THIAZOLE EN TANT QU'ANTAGONISTES DE A2B

(30) Priority: 21.01.2004 GB 0401336
(43) Date of publication of application: 11.10.2006
(73) Proprietor: Novartis AG, 4056 Basel (CH); NOVARTIS-PHARMA GMBH, 1230 Vienna (AT)
(72) Inventor: PRESS, Neil John, Novartis Horsham Research Cntr, Horsham, West Sussex RH12 5AB (GB); TAYLOR, Roger J., Novartis Horsham Research Cntr, Horsham, West Sussex RH12 5AB (GB)
(86) International application number: PCT/EP2005/000542
(87) International publication number: WO 2005/070926

(56) References cited:
- WO-A-02/42298
- WO-A-99/64418
- WO-A-03/039451

## Description

This invention relates to organic compounds, their preparation and their use as pharmaceuticals.

WO 99/64418, WO 03/039451 and WO 02/422 98 all disclose thiazole derivatives having affinity for adenosine receptors.

In one aspect, the present invention provides compounds of formula 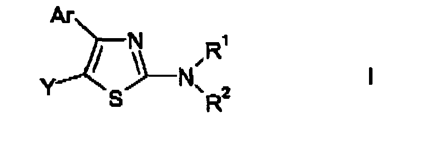 in free or salt form, where
Ar is phenyl substituted by one or more substituents selected from halogen, cyano and C₁-C₈-haloalkyl, or naphthyl,
R¹ is hydrogen, phenyl optionally substituted by one or more substituents selected from halogen, cyano, hydroxy, C₁-C₈-alkyl, C₁-C₈-haloalkyl, C₁-C₈-alkoxy, C₁-C₈-alkoxy-C₁-C₈-alkyl, carboxy, C₁-C₈-alkoxycarbonyl and acyloxy, or R¹ is a 5- or 6- membered monovalent heterocyclic group,
R² is hydrogen, C₁-C₈-alkyl, acyl or -CON(R³)R⁴,
R³ and R⁴ are each independently hydrogen or C₁-C₈-alkyl, or together with the nitrogen atom to which they are attached denote a 5- or 6- membered heterocyclic group, and
Y is a pyrimidinyl or pyridazinyl group, optionally substituted by at least one C₁-C₈-alkyl, C₁-C₈-alkoxy, C₁-C₈-alkylthio, C₁-C₈-alkylamino, di(C₁-C₈-alkyl)amino or acylamino group.

Terms used in the specification have the following meanings :
"C₁-C₈-alkyl" as used herein denotes straight chain or branched C₁-C₈-alkyl, which may be, for example, methyl, ethyl, n-propyl, isopropyl, n-butyl, isobutyl, sec-buryl, tert-butyl, straight or branched pentyl, straight or branched hexyl, straight or branched heptyl, or straight or branched octyl. Preferably, C₁-C₈-alkyl is C₁-C₄-alkyl.
"C₁-C₈-alkoxy" as used herein denotes straight chain or branched C₁-C₈-alkoxy which may be, for example, methoxy, ethoxy, n-propoxy, isopropoxy, n-butoxy, isobutoxy, sec-buroxy, tert-buroxy, straight or branched pentoxy, straight or branched hexyloxy, straight or branched heptyloxy, or straight or branched octyloxy. Preferably, C₁-C₈-alkoxy is C₁-C₄-alkoxy.
"C₁-C₈-alkylthio" as used herein denotes straight chain or branched C₁-C₈-alkylthio which may be, for example, methylthio, ethylthio, n-propylthio, isopropylthio, n-butylthio, isobutylthio, sec-butylthio, tert-butylthio, straight or branched pentylthio, straight or branched hexylthio, straight or branched heptylthio, or straight or branched octylthio. Preferably, C₁-C₈-alkylthio is C₁-C₄-alkylthio.
"di(C₁-C₈-alkyl)amino"" as used herein denotes amino substituted by two C₁-C₈-alkyl groups as hereinbefore defined, which may be the same or different. Preferably, di(C₁-C₈-alkyl)amino is di(C₁-C₄-alkyl)amino.
"C₁-C₈-haloalkyl" as used herein denotes C₁-C₈-alkyl as hereinbefore defined substituted by one or more halogen atoms, preferably one, two or three halogen atoms, preferably fluorine or chlorine atoms. Preferably C₁-C₈-haloalkyl is C₁-C₄-alkyl substituted by one, two or three fluorine or chlorine atoms.
"C₁-C₈-alkoxy-C₁-C₈-alkyl" as used herein denotes C₁-C₈-alkyl as hereinbefore defined substituted by C₁-C₈-alkoxy as hereinbefore defined.
"C₁-C₈-alkylcarbonyl", "C₁-C₈-haloalkylcarbonyl" and "C₁-C₈-alkoxycarbonyl" as used herein denote C₁-C₈-alkyl, C₁-C₈-haloalkyl or C₁-C₈-alkoxy respectively as hereinbefore defined attached by a carbon atom to a carbonyl group.
"Acyl" as used herein denotes alkylcarbonyl, for example C₁-C₈-alkylcarbonyl where C₁-C₈-alkyl may be one of the C₁-C₈-alkyl groups hereinbefore mentioned, optionally substituted by one or more halogen atoms; cycloalkylcarbonyl, for example C₃-C₈-cycloalkylcarbonyl where C₃-C₈-cycloalkyl may be, for example, cyclopropyl, cyclobutyl, cyclopentyl, cyclohexyl, cycloheptyl or cyclooctyl; 5- or 6- membered heterocyclylcarbonyl having one or more, preferably one or two, hetero atoms selected from nitrogen, oxygen and sulfur in the ring, such as furylcarbonyl or pyridylcarbonyl; arylcarbonyl, for example C₆-C₁₀-arylcarbonyl such as benzoyl; or aralkylcarbonyl, for example C₆ to C₁₀-aryl-C₁-C₄-alkylcarbonyl such as benzylcarbonyl or phenylethylcarbonyl.
"Acyloxy" as used herein denotes alkylcarbonyloxy, for example C₁-C₈-alkylcarbonyloxy where C₁-C₈-alkyl may be one of the C₁-C₈-alkyl groups hereinbefore mentioned, optionally substituted by one or more halogen atoms; cycloalkylcarbonyloxy, for example C₃-C₈-cycloalkylcarbonyloxy where C₃-C₈-cycloalkyl may be, for example, cyclopropyl, cyclobutyl, cyclopentyl, cyclohexyl, cycloheptyl or cyclooctyl; 5- or 6- membered heterocyclylcarbonyloxy having one or two hetero atoms selected from nitrogen, oxygen and sulfur in the ring, such as furylcarbonyloxy or pyridylcarbonyloxy; arylcarbonyloxy, for example C₆-C₁₀-arylcarbonyloxy such as benzoyloxy; or aralkylcarbonyloxy, for example C₆ to C₁₀-aryl-C₁-C₄-alkylcarbonyloxy such as benzylcarbonyloxy or phenylethylcarbonyloxy. Preferably acyloxy is C₁-C₄-alkylcarbonyloxy.
"Acylamino" as used herein denotes amino substituted by acyl as hereinbefore defined. Preferably it is C₁-C₄-alkylcarbonylamino.
"Halogen" as used herein may be fluorine, chlorine, bromine or iodine; preferably it is fluorine or chlorine.

Ar may be, for example, phenyl substituted by one or more substituents, for example, one, two or three substituents selected from halogen, cyano and C₁-C₈-haloalkyl, or naphthyl. Ar is preferably phenyl substituted by halogen or cyano, preferably meta or para to the indicated thiazole ring.

R¹ may be, for example, hydrogen, phenyl optionally substituted by halogen, cyano, hydroxy, C₁-C₈-alkyl, C₁-C₈-haloalkyl, C₁-C₈-alkoxy, C₁-C₈-alkoxy-C₁-C₈-alkyl, carboxy, C₁-C₈-alkoxycarbonyl or C₁-C₈-alkylcarbonyloxy, or a monovalent 5- or 6- membered heterocyclic group having one, two or three ring hetero atoms selected from nitrogen, oxygen and sulfur, such as pyrrolyl, imidazolyl, triazolyl, pyridyl, oxopyridyl, piperidyl, pyridazinyl, pyrimidinyl, pyrazinyl, pyrazolyl, pyrazolinyl, piperazinyl, morpholinyl, furyl, pyranyl, thienyl or thiazolyl, optionally substituted by one or more substituents selected from C₁-C₈-alkyl, hydroxy or C₁-C₈-alkoxy. Preferably R¹ is phenyl optionally substituted by cyano, carboxy or C₁-C₄-alkoxy, or a monovalent 6-membered N-heterocyclic group, especially pyridyl, C₁-C₄-alkylpyridyl, C₁-C₄-alkoxypyridyl or pyrazinyl.

R² may be, for example, hydrogen, C₁-C₈-alkyl, formyl, C₁-C₈-alkylcarbonyl, C₁-C₈-haloalkylcarbonyl, C₃-C₈-cycloalkylcarbonyl, phenylcarbonyl in which the phenyl moiety is optionally substituted by halogen, cyano, hydroxy, C₁-C₈-alkyl or C₁-C₈-alkoxy, heterocyclylcarbonyl in which the heterocyclyl group is 5- or 6-membered and has one or more, preferably one or two, ring hetero atoms selected from nitrogen, oxygen and sulfur, or a group -CON(R³)R⁴. Preferably R² is hydrogen, C₁-C₄-alkylcarbonyl, 5-membered heterocyclylcarbonyl, especially furylcarbonyl, or phenylcarbonyl in which the phenyl moiety is optionally substituted by C₁-C₈-alkoxy, especially C₁-C₄-alkoxyphenylcarbonyl.

Where present, R³ and R⁴ may each independently be, for example, hydrogen or C₁-C₄-alkyl, or together with the nitrogen atom to which the are attached may denote a 5-membered heterocyclyl group such as pyrrolyl or pyrrolidinyl or a 6-membered heterocyclyl group such as pyridyl, piperidyl, piperazinyl or morpholinyl. Preferably R³ and R⁴, where present, are each C₁-C₈-alkyl, especially methyl, or together with the nitrogen atom to which they are attached denote a 6-membered heterocyclyl group, especially pyridyl.

Y may be, for example, a pyrimidinyl group of formula 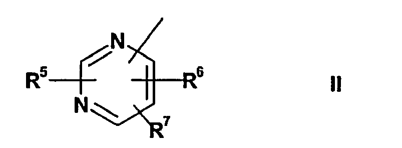 where R⁵, R⁶ and R⁷ are each independently hydrogen, C₁-C₈-alkyl, C₁-C₈-alkoxy, C₁-C₈-alkylthio, C₁-C₈-alkylamino, di(C₁-C₈-alkylamino or acylamino, or Y may be a group of formula 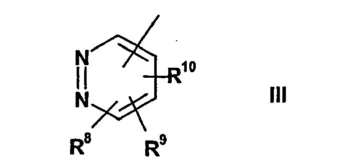 where R⁸, R⁹ and R¹⁰ are each independently hydrogen, C₁-C₈-alkyl, C₁-C₈-alkoxy, C₁-C₈-alkylthio, C₁-C₈-alkylamino, di(C₁-C₈-alkyl) amino or acylamino.

Preferably Y is a group of formula 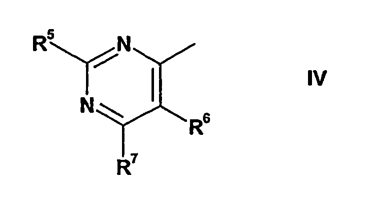 where R⁵ and R⁶ are each hydrogen and R⁷ is hydrogen, C₁-C₄-alkyl, C₁-C₄-alkylthio, or Y is a group of formula 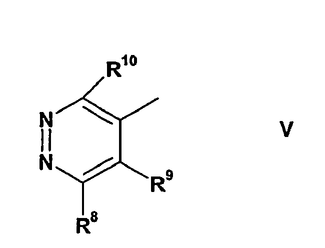 where R⁹ and R¹⁰ are each hydrogen and R⁸ is hydrogen or di(C₁-C₄-alkyl) amino.

Preferred compounds of formula I in free or salt form are those where
Ar is phenyl substituted by halogen or cyano,
R¹ is hydrogen, phenyl optionally substituted by cyano, halogen, carboxy or C₁-C₄-alkoxy, or R¹ is a monovalent 6-membered N-heterocyclic group,
R² is hydrogen, C₁-C₄-alkylcarbonyl, 5-membered heterocyclylcarbonyl or phenylcarbonyl in which the phenyl moiety is optionally substituted by C₁-C₈-alkoxy, and
Y is pyrimidinyl or pyridazinyl optionally substituted by C₁-C₄-alkyl, C₁-C₄-alkoxy, C₁-C₄-alkylthio, C₁-C₄-alkylamino, di(C₁-C₄-alkyl) amino or C₁-C₄-alkylcarbonylamino.
Further preferred compounds of formula I in free or salt form are those where
Ar is phenyl substituted by cyano meta to the indicated thiazole ring,
R¹ is hydrogen, phenyl substituted by cyano, fluorine, carboxy or C₁-C₄-alkoxy or R¹ is 6-membered N-heterocyclyl having one or two ring nitrogen atoms, optionally substituted by C₁-C₄-alkyl or C₁-C₄-alkoxy,
R² is hydrogen, C₁-C₄-alkylcarbonyl, furylcarbonyl or C₁-C₄-alkoxyphenylcarbonyl, and
Y is a group of formula IV or V as hereinbefore defined.

Especially preferred specific compounds of formula I are those described hereinafter in the Examples.

Many of the compounds represented by formula I are capable of forming acid addition salts, particularly pharmaceutically acceptable acid addition salts. Pharmaceutically acceptable acid addition salts of the compound of formula I include those of inorganic acids, for example, hydrohalic acids such as hydrofluoric acid, hydrochloric acid, hydrobromic acid or hydroiodic acid, nitric acid, sulfuric acid, phosphoric acid; and organic acids, for example aliphatic monocarboxylic acids such as formic acid, acetic acid, trifluoroacetic acid, propionic acid and butyric acid, aliphatic hydroxy acids such as lactic acid, citric acid, tartaric acid or malic acid, dicarboxylic acids such as maleic acid or succinic acid, aromatic carboxylic acids such as benzoic acid, p-chlorobenzoic acid, diphenylacetic acid or triphenylacetic acid, aromatic hydroxy acids such as o-hydroxybenzoic acid, p-hydroxybenzoic acid, 1-hydroxynaphthalene-2-carboxylic acid or 3-hydroxynaphthalene-2-carboxylic acid, and sulfonic acids such as methanesulfonic acid or benzenesulfonic acid. These salts may be prepared from compounds of formula I by known salt-forming procedures.

Compounds of formula I which contain acidic, e.g. carboxyl, groups, are also capable of forming salts with bases, in particular pharmaceutically acceptable bases such as those well known in the art; suitable such salts include metal salts, particularly alkali metal or alkaline earth metal salts such as sodium, potassium, magnesium or calcium salts, or salts with ammonia or pharmaceutically acceptable organic amines or heterocyclic bases such as ethanolamines, benzylamines or pyridine. These salts may be prepared from compounds of formula I by known salt-forming procedures.

The invention provides, in another aspect, a method of preparing a compound of formula I in free or salt form which comprises
(i)
   (A) for the preparation of compounds of formula I where R¹ is optionally substituted phenyl or a 5- or 6- membered heterocyclic group, reacting a compound of formula 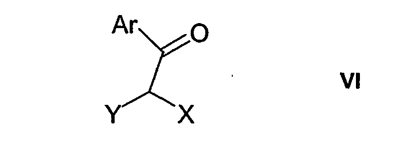 in the form of a salt, e.g. a hydrohalide salt thereof, where Ar and Y are as hereinbefore defined and X is halogen, preferably bromine, with a compound of formula 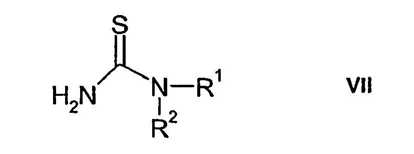 where R¹ is phenyl optionally substituted by one or more substituents selected from halogen, cyano, hydroxy, C₁-C₈-alkyl, C₁-C₈-haloalkyl, C₁-C₈-alkoxy, C₁-C₈-alkoxy-C₁-C₈-alkyl and acyloxy or R¹ is a 5- or 6- membered monovalent heterocyclic group, and R² is H or C₁-C₈-alkyl or
   (B) for the preparation of compounds of formula I where R² is acyl or -CON(R³)R⁴, reacting a compound of formula 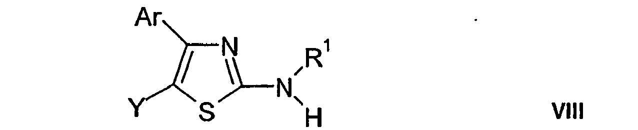 where Ar, R¹ and Y are as hereinbefore defined with, respectively, an acylating derivative of a carboxylic acid, for example the anhydride or acid chloride thereof, or with a compound of formula Cl-CON(R³)R⁴) where R³ and R⁴ are as hereinbefore defined, and
(ii) recovering the resultant compound of formula I in free or salt form.

Process variant (A) may be carried out in an organic solvent, for example an alcohol such as ethanol. Suitable reaction temperatures are elevated temperatures, for example from 50°C to reflux temperature of the solvent.

Process variant (B) may be carried out using known procedures for reaction of amines with acylating agents.

Compounds of formula VI may be prepared by reacting a compound of formula 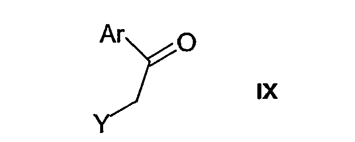 where Ar and Y are as hereinbefore defined, with halogen X₂, preferably bromine. This halogenation may be effected using known procedures for alpha halogenation of ketones. Conveniently, the compound of formula VI is not isolated but is reacted directly with a compound of formula VII to give a compound of formula I.

Compounds of formula VII are thioureas which are either known or may be obtained by known procedures. For example they may be prepared by reaction of a compound of formula 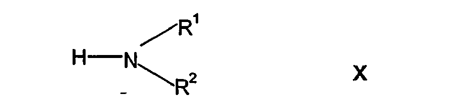 where R¹ and R² are as hereinbefore defined, with benzoyl isothiocyanate and hydrolysing the resulting product, for example with aqueous NaOH, to replace the benzoyl group by halogen. The reaction with benzoyl isothiocyanate may be carried out in an organic solvent, for example an alcohol such as ethanol. Suitable reaction temperatures are from room temperature to reflux temperature of the solvent, conveniently 35-45°C. The hydrolysis may be effected at elevated temperature, for example 70°C to reflux temperature, conveniently at reflux temperature.

Pyrimidine, pyridazine and alkyl-substituted pyrimidine and pyridazine are known compounds which are commercially available or may be prepared by known procedures. Compounds of formula VIII may be prepared by process variant (A) as described above. Compounds of formula IX may be prepared by reaction of a compound of formula 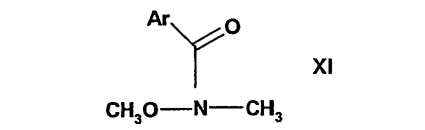 with an alkali metal derivative of the pyrimidine or pyridazine, of formula MCH₂Y where M is an alkali metal, preferably lithium or sodium, and Y is as hereinbefore defined, the CH₂M group preferably being attached to the 4-position in the pyrimidinyl or pyridazinyl group, e.g. using a known procedure such as described hereinafter in the Examples. Compounds of fomula X and XI are known or may be obtained by known procedures. For example, compounds of formula XI may be prepared by procedures such as described hereinafter in the Examples.

Compounds of formula I and their pharmaceutically acceptable salts are useful as pharmaceuticals. In particular, they exhibit inhibition of adenosine A2b receptor activation, i.e. they act as A2b receptor antagonists. Moreover, in general they selectively inhibit activation of A2b receptor over the adenosine A1 and A2a receptors. Their inhibitory properties may be demonstrated in the following test procedures:

### Adenosine A2b Receptor Reporter Gene Assay

### a) Culturing of Chinese Hamster Ovary (CHO) A2b Cell Line

CHO cells transfected with a Luciferase-expressing reporter plasmid (pCRE-LUCI) and with a plasmid carrying the human adenosine A2b receptor structural gene (pA2bRCV) are routinely cultured in Dulbecco's Modified Eagle Medium (DMEM) - supplemented with 10% v/v fetal calf serum (FCS), 2mM L-glutamine, 0.4 mg/ml L-proline, 1 nM sodium selenite, 0.5 mg/ml Hygromycin B and 1 mg/ml Geneticin - at 37°C, 5% CO₂, and 100% humidity. The cells are left to grow to confluence for 4-5 days. The cells obtained are passaged using trypsin/EDTA and split at a ratio of 1 in 5.

### b) Preparation of cells for assay

Prior to the assay, the CHO-A2b cells are plated onto white 96-well View Plate tissue culture plates (Packard) at a density of 50,000 cells per well in 50 µl of DMEM, and the plates are incubated at 37°C, 5% CO₂ and 100 % humidity.

### c) Preparation of Reference and Test Compounds

10 mM solutions of the reference compound, Xanthine Amine Cogener (XAC), and the test compound in dimethyl sulfoxide (DMSO) are prepared. The solutions are further diluted with DMSO to 100 µM, then diluted to 10 µM, and finally to 250 nM or 2.5 µM with Assay Buffer (DMEM Phenol Red-free tissue culture media supplemented with 10 µM Rolipram and 10 U/ml adenosine deaminase (ADA). The resulting solutions (40 µl) are added to the cells in the appropriate wells, the final concentration per well being 100 nM or 1 µM, and the plates are incubated at 37°C, 5% CO₂ and 100% humidity.

### d) Luciferase Reporter Gene Assay

5'-N-ethylcarboxamidoadenosine (NECA), an adenosine A2b agonist, is prepared as a 10 nM solution in DMSO and then diluted to 100 µM with Assay Buffer. This solution is serially diluted in Assay Buffer to give a series of 10 NECA concentrations from 100 to 0.01 µM. 10 µl portions of the resulting NECA solutions are added to the mixtures of CHO-A2b cells and reference or test compound solutions prepared as described above (preincubated for 30 minutes), final concentrations ranging from 10 to 0.0005 µM per well. The cells are incubated at 37°C, 5% CO₂ and 100% humidity for 3 hours to induce release of cAMP, which then binds to cAMP binding protein (CBP) and the resulting complex interacts with the reporter plasmid to express Luciferase. 100 µl of Steady-Glo, a Luciferase assay substrate from Promega, is added to all wells to lyse the cells and generate luminescence in proportion to the amount of Lucifrease produced. The plates are left for a minimum of 5 minutes before being read on the luminescence program of a Topcount NXT microplate scintillation counter (ex Packard). Concentration - response curves are plotted from the luminescence data using Activitybase software and K_{B} values for the antagonists under test are calculated from the shifts of the curve at a particular concentration (K_{B} = [antagonist]/(concentration ratio -1)

Compounds of the Examples hereinbelow have K_{B} values below 100nM in the reporter gene assay. For example, the compounds of Examples 4,6,9,12 and 16 have K_{B} values of 38, 29, 6, 2 and 50nM respectively.

Having regard to their inhibition of adenosine A2b receptor activation, compounds of formula I in free or pharmaceutically acceptable salt form, hereinafter alternately referred to as agents of the invention, are useful in the treatment of conditions which are mediated by the activation of the adenosine A2b receptor, particularly inflammatory or allergic conditions. Treatment in accordance with the invention may be symptomatic or prophylactic.

Accordingly, agents of the invention are useful in the treatment of inflammatory or obstructive airways diseases, resulting, for example, in reduction of tissue damage, bronchial hyperreactivity, remodelling or disease progression. Inflammatory or obstructive airways diseases to which the present invention is applicable include asthma of whatever type or genesis including both intrinsic (non-allergic) asthma and extrinsic (allergic) asthma, mild asthma, moderate asthma, severe asthma, bronchitic asthma, excercise-induced asthma, occupational asthma and asthma induced following bacterial infection. Treatment of asthma is also to be understood as embracing treatment of subjects, e.g. of less than 4 or 5 years of age, exhibiting wheezing symptoms and diagnosed or diagnosable as "wheezy infants", an established patient category of major medical concern and now often identified as incipient or early-phase asthmatics. (For convenience this particular asthmatic condition is referred to as "wheezy-infant syndrome".)

Prophylactic efficacy in the treatment of asthma will be evidenced by reduced frequency or severity of symptomatic attack, e.g. of acute asthmatic or bronchoconstrictor attack, improvement in lung function or improved airways hyperreactivity. It may further be evidenced by reduced requirement for other, symptomatic therapy, i.e. therapy for or intended to restrict or abort symptomatic attack when it occurs, for example anti-inflammatory (e.g. corticosteroid) or bronchodilatory. Prophylactic benefit in asthma may in particular be apparent in subjects prone to "morning dipping". "Morning dipping" is a recognised asthmatic syndrome, common to a substantial percentage of asthmatics and
characterised by asthma attack, e.g. between the hours of about 4 to 6 am, i.e. at a time normally substantially distant form any previously administered symptomatic asthma therapy.

Other inflammatory or obstructive airways diseases and conditions to which the present invention is applicable include acute lung injury (ALI), adult respiratory distress syndrome (ARDS), chronic obstructive pulmonary, airways or lung disease (COPD, COAD or COLD), including chronic bronchitis or dyspnea associated therewith, emphysema, as well as exacerbation of airways hyperreactivity consequent to other drug therapy, in particular other inhaled drug therapy. The invention is also applicable to the treatment of bronchitis of whatever type or genesis including, e.g., acute, arachidic, catarrhal, croupus, chronic or phthinoid bronchitis. Further inflammatory or obstructive airways diseases to which the present invention is applicable include pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tabacosis and byssinosis.

Having regard to their anti-inflammatory activity, in particular in relation to inhibition of eosinophil activation, agents of the invention are also useful in the treatment of eosinophil related disorders, e.g. eosinophilia, in particular eosinophil related disorders of the airways (e.g. involving morbid eosinophilic infiltration of pulmonary tissues) including hypereosinophilia as it effects the airways and/or lungs as well as, for example, eosinophil-related disorders of the airways consequential or concomitant to Löffler's syndrome, eosinophilic pneumonia, parasitic (in particular metazoan) infestation (including tropical eosinophilia), bronchopulmonary aspergillosis, polyarteritis nodosa (including Churg-Strauss syndrome), eosinophilic granuloma and eosinophil-related disorders affecting the airways occasioned by drug-reaction.

Agents of the invention are also useful in the treatment of inflammatory or allergic conditions of the skin, for example psoriasis, contact dermatitis, atopic dermatitis, alopecia areata, erythema multiforma, dermatitis herpetiformis, scleroderma, vitiligo, hypersensitivity angiitis, urticaria, bullous pemphigoid, lupus erythematosus, pemphisus, epidermolysis bullosa acquisita, and other inflammatory or allergic conditions of the skin.

Agents of the invention may also be used for the treatment of other diseases or conditions, in particular diseases or conditions having an inflammatory component, for example, treatment of diseases and conditions of the eye such as conjunctivitis, keratoconjunctivitis sicca, and vernal conjunctivitis, diseases affecting the nose including allergic rhinitis, and inflammatory disease in which autoimmune reactions are implicated or having an autoimmune component or aetiology, including autoimmune haematological disorders (e.g. haemolytic anaemia, aplastic anaemia, pure red cell anaemia and idiopathic thrombocytopenia), systemic lupus erythematosus, polychondritis, sclerodoma, Wegener granulamatosis, dermatomyositis, chronic active hepatitis, myasthenia gravis, Steven-Johnson syndrome, idiopathic sprue, autoimmune inflammatory bowel disease (e.g. ulcerative colitis and Crohn's disease), endocrine opthalmopathy, Grave's disease, sarcoidosis, alveolitis, chronic hypersensitivity pneumonitis, multiple sclerosis, primary billiary cirrhosis, diabetes, juvenile diabetes (diabetes mellitus type I), uveitis (anterior and posterior), keratoconjunctivitis sicca and vernal keratoconjunctivitis, interstitial lung fibrosis, psoriatic arthritis and glomerulonephritis (with and without nephrotic syndrome, e.g. including idiopathic nephrotic syndrome or minal change nephropathy).

Other diseases or conditions mediated by the adenosine A2b receptor which may be treated with agents of the invention include diarrheal diseases, ischemia/reperfusion injuries or retinopathy such as diabetic retinopathy or hyperbaric oxygen-induced retinopathy.

The effectiveness of an agent of the invention in inhibiting inflammatory conditions, for example in inflammatory airways diseases, may be demonstrated in an animal model, e.g. a mouse or rat model, of airways inflammation or other inflammatory conditions, for example as described by Szarka et al, J. Immunol. Methods (1997) 202:49-57; Renzi et al, Am. Rev. Respir. Dis. (1993) 148:932-939; Tsuyuki et al., J. Clin. Invest. (1995) 96:2924-2931; Cernadas et al (1999) Am. J. Respir. Cell Mol. Biol. 20:1-8; and Fozard et al (2002) European Journal of Pharmacological 438, 183-188.

The agents of the invention are also useful as co-therapeutic agents for use in combination with other drug substances such as anti-inflammatory, bronchodilatory, antihistamine or anti-tussive drug substances, particularly in the treatment of obstructive or inflammatory airways diseases such as those mentioned hereinbefore, for example as potentiators of therapeutic activity of such drugs or as a means of reducing required dosaging or potential side effects of such drugs. An agent of the invention may be mixed with the other drug substance in a fixed pharmaceutical composition or it may be administered separately, before, simultaneously with or after the other drug substance.

Accordingly the invention includes a combination of an agent of the invention as hereinbefore described with an anti-inflammatory, bronchodilatory, antihistamine or anti-tussive drug substance, said agent of the invention and said drug substance being in the same or different pharmaceutical composition.

Suitable anti-inflammatory drugs include steroids, in particular glucocorticosteroids such as budesonide, beclamethasone dipropionate, fluticasone propionate, ciclesonide or mometasone furoate, or steroids described in WO 02/88167, WO 02/12266, WO 02/100879, WO 02/00679 (especially those of Examples 3,11,14,17,19, 26, 34, 37, 39, 51, 60, 67, 72, 73, 90, 99 and 101), WO 03/35668, WO 03/48181, WO 03/62259, WO 03/64445, WO 03/72592, WO 04/39827 and WO 04/66920; non-steroidal glucocorticoid receptor agonists, such as those described in DE 10261874, WO 00/00531, WO 02/10143, WO 03/82280, WO 03/82787, WO 03/86294, WO 03/104195, WO 03/101932, WO 04/05229, WO 04/18429, WO 04/19935 and WO 04/26248; LTD4 antagonists such as montelukast and zafirlukast; PDE4 inhibitors such cilomilast (Ariflo^{®} GlaxoSmithKline), Roflumilast (Byk Gulden),V-11294A (Napp), BAY19-8004 (Bayer), SCH-351591 (Schering-Plough), Arofylline (Almirall Prodesfarma), PD189659 / PD168787 (Parke-Davis), AWD-12-281 (Asta Medica), CDC-801 (Celgene), SelCID(TM) CC-10004 (Celgene), VM554/UM565 (Vernalis), T-440 (Tanabe), KW-4490 (Kyowa Hakko Kogyo), and those disclosed in WO 92/19594, WO 93/19749, WO 93/19750, WO 93/19751, WO 98/18796, WO 99/16766, WO 01/13953, WO 03/104204, WO 03/104205, WO 03/39544, WO 04/000814, WO 04/000839, WO 04/005258, WO 04/018450, WO 04/018451, WO 04/018457, WO 04/018465, WO 04/018431, WO 04/018449, WO 04/018450, WO 04/018451, WO 04/018457, WO 04/018465, WO 04/019944, WO 04/019945, WO 04/045607 and WO 04/037805; and adenosine A_{2A} receptor agonists such as those described in WO 98/28319, WO 99/67265 and WO 01/094368.

Suitable bronchodilatory drugs include a) anticholinergic or antimuscarinic agents, in particular ipratropium bromide, oxitropium bromide, tiotropium salts and CHF 4226 (Chiesi), and glycopyrrolate, but also those described in EP 424021, US 3714357, US 5171744, WO 01/04118, WO 02/00652, WO 02/51841, WO 02/53564, WO 03/00840, WO 03/33495, WO 03/53966, WO 03/87094, WO 04/018422 and WO 04/05285; and b) beta-2 adrenoceptor agonists such as albuterol (salbutamol), metaproterenol, terbutaline, salmeterol fenoterol, procaterol, and especially, formoterol, carmoterol and pharmaceutically acceptable salts thereof, and compounds (in free or salt or solvate form) of formula I of WO 0075114, which document is incorporated herein by reference, preferably compounds of the Examples thereof, especially a compound of formula 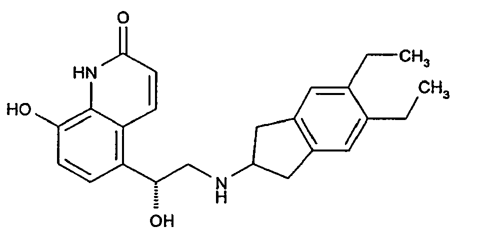 and pharmaceutically acceptable salts thereof, as well as compounds (in free or salt or solvate form) of formula I of WO 04/16601, and also compounds of EP 1440966, JP 05025045, WO 93/18007, WO 99/64035, US 2002/0055651, WO 01/42193, WO 01/83462, WO 02/66422, WO 02/ 70490, WO 02/76933, WO 03/24439, WO 03/42160, WO 03/42164, WO 03/72539, WO 03/91204, WO 03/99764, WO 04/16578, WO 04/22547, WO 04/32921, WO 04/33412, WO 04/37768, WO 04/37773, WO 04/37807, WO 04/39762, WO 04/39766, WO 04/45618 WO 04/46083 and WO 04/80964.

Suitable dual acting bronchodilatory drugs include dual beta-2 adrenoceptor agonist /muscarinic antagonists such as those disclosed in US 2004/0167167, WO 04/74246 and WO 04/74812.

Suitable antihistamine drug substances include cetirizine hydrochloride, acetaminophen, clemastine fumarate, promethazine, loratidine, desloratidine, diphenhydramine and fexofenadine hydrochloride, activastine, astemizole, azelastine, ebastine, epinastine, mizolastine and tefenadine as well as those disclosed in JP 2004107299, WO 03/099807 and WO 04/026841.

Other useful combinations of agents of the invention with anti-inflammatory drugs are those with antagonists of chemokine receptors, e.g. CCR-1, CCR-2, CCR-3, CCR-4, CCR-5, CCR-6, CCR-7, CCR-8, CCR-9 and CCR10, CXCR1, CXCR2, CXCR3, CXCR4, CXCR5, particularly CCR-5 antagonists such as Schering-Plough antagonists SC-351125, SCH-55700 and SCH-D, Takeda antagonists such as N-[[4-[[[6,7-dihydro-2-(4-methylphenyl)-5H-benzo-cyclohepten-8-yl]carbonyl]amino]phenyl]-methyl]tetrahydro-N,N-dimethyl-2H-pyran-4-amin-ium chloride (TAK-770), and CCR-5 antagonists described in US 6166037 (particularly claims 18 and 19), WO 00/66558 (particularly claim 8), WO 00/66559 (particularly claim 9), WO 04/018425 and WO 04/026873.

The compounds of the invention can be used in a method for the treatment of a condition mediated by activation of the adenosine A2b receptor, for example an inflammatory or allergic condition, particularly an inflammatory or obstructive airways disease, which comprises administering to a subject, particularly a human subject, in need thereof an effective amount of a compound of formula I in free form or in the form of a pharmaceutically acceptable salt. In another aspect the invention provides a compound of formula I, in free form or in the form of a pharmaceutically acceptable salt, for use in the manufacture of a medicament for the treatment of a condition mediated by activation of the adenosine A2b receptor, particularly an inflammatory or obstructive airways disease.

The agents of the invention may be administered by any appropriate route, e.g. orally, for example in the form of a tablet or capsule, for example in the treatment of inflammatory or obstructive airways disease; parenterally, for example intravenously; by inhalation, for example in the treatment of inflammatory or obstructive airways disease; intranasally, for example in the treatment of allergic rhinitis; topically to the skin, for example in the treatment of atopic dermatitis; or rectally, for example in the treatment of inflammatory bowel disease.

In a further aspect, the invention also provides a pharmaceutical composition comprising a compound of formula I in free form or in the form of a pharmaceutically acceptable salt, optionally together with a pharmaceutically acceptable diluent or carrier therefor. The composition may contain a co-therapeutic agent such as an anti-inflammatory, bronchodilatory, antihistamine or anti-tussive drug substance as hereinbefore described. Such compositions may be prepared using conventional diluents or excipients and techniques known in the galenic art. Thus oral dosage forms may include tablets and capsules. Formulations for topical administration may take the form of creams, ointments, gels or transdermal delivery systems, e.g. patches. Compositions for inhalation may comprise aerosol or other atomizable formulations or dry powder formulations.

Dosages of agents of the invention employed in practising the present invention will of course vary depending, for example, on the particular condition to be treated, the effect desired and the mode of administration. In general, suitable daily dosages for oral administration are of the order of 0.1 to 10 mg/kg.

The invention is illustrated by the following Examples.

### Examples 1-12

Compounds of formula I which are also of formula 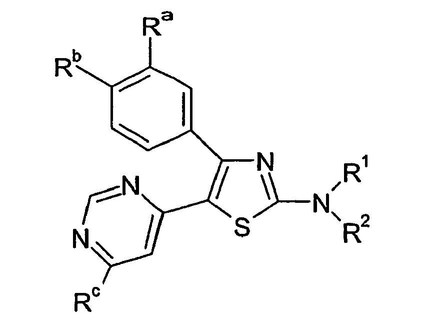 are shown in the following table, the method of preparation being described hereinafter. The table also shows mass spectrometry data. The Examples are in free form.

| Ex. No. | R^{a} | R^{b} | R^{c} | R¹ | R² | m/s (MH+) |
|---|---|---|---|---|---|---|
| 1 | CN | H | CH₃ | 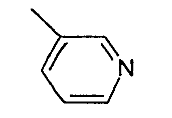 | H | 371.2 |
| 2 | CN | H | H | 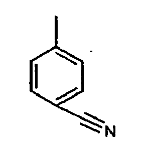 | H | 381 |
| 3 | CN | H | H | 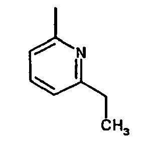 | H | 385.5 |
| 4 | CN | H | H | 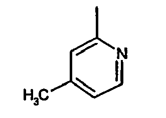 | H | 371.4 |
| 5 | CN | H | CH₃ | 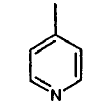 | H | 371.2 |
| 6 | CN | H | H | 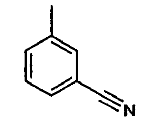 | H | 381 |
| 7 | CN | H | H | 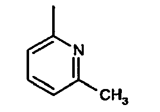 | H | 371 |
| 8 | CN | H | H | 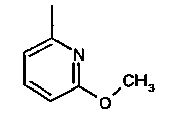 | H | 387.5 |
| 9 | CN | H | H | 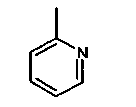 | H | 357.1 |
| 10 | CN | H | H | H | 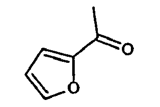 | 374.1 |
| 11 | CN | H | CH₃ | H | 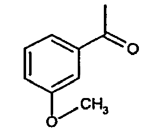 | 414 |
| 12 | CN | H | H | 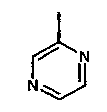 | H | 358 |

### Examples 13-16

Compounds of formula I which are also of formula 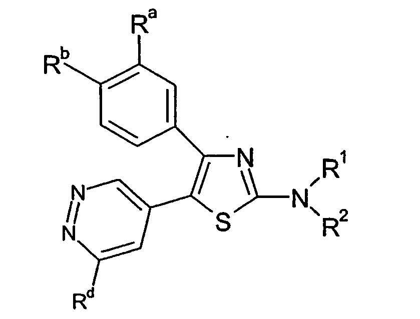 are shown in the following table, the method of preparation being described hereinafter. The table also shows mass spectrometry data. The Examples are in free form, with the exception of Example 16 which is in the form of a salt with trifluoroacetic acid.

| Ex. | R^{a} | R^{b} | R^{d} | R¹ | R² | M/s MH+ |
|---|---|---|---|---|---|---|
| 13 | CN | H | H | H | COCH₃ | 322.4 |
| 14 | CN | H | H | 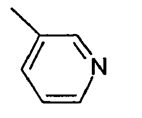 | H | 357.4 |
| 15 | CN | H | H | 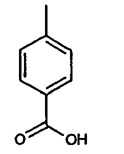 | H | 400.4 |
| 16 | CN | H | N(CH₃)₂ | 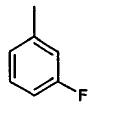 | H | 417.5 |

### Preparation of 3-cyano-N-methoxy-N-methylbenzamide

3-cyanobenzoic acid (7.00g, 47.6mmol) is dissolved in THF (50ml) under nitrogen and N,N'-carbonylbisimidazole (7.95g, 49.0mmol) is added. The mixture is stirred under reflux for 25mins, then cooled and dimethylhydroxylamine hydrochloride (4.78g, 49.0mmol) is added. The mixture is heated under reflux for 5 hours and then stood at room temperature for 18h. The reaction mixture is added to water (200ml). The aqueous mixture is extracted with ether twice, and the combined ether phases are washed with saturated aqueous sodium carbonate. The organic layer is dried (MgSO₄) and concentrated to a colourless mobile oil. MS: (APCI) MH⁺ at 191

### Preparation of compounds of formula IX

### 3-(Pyridazin-4-yl-acetyl)-benzonitrile

A solution of diisopropylamine (4.08ml, 29.0mmol) in dry THF (35ml) is cooled to -70°C and 1.6M BuLi in hexanes (18.1ml, 29.0mmol) is added dropwise. The cooling bath is then removed and the mixture is allowed to warm to 0°C. The solution is recooled to -70°C and 4-methylpyridazine (2.5g, 26.6mmol) is added dropwise, and the solution stirred at -70°C for 1.75h. To the resulting brown solution 3-cyano-N-methoxy-N-methyl-benzamide (5.06g, 26.6mmol) in dry tetrahydrofuran (10ml) is added dropwise. The solution is stirred at -70°C for 1h, then warmed to -30°C. After 1h the mixture is allowed to warm to room temperature. Saturated aqueous ammonium chloride (20ml) is added to the reaction, and the solvent is removed by evaporation to yield a yellow solid, which is washed with aqueous sodium bicarbonate, water and then ethyl acetate. The product is obtained as a yellow solid, m.p. 142-144°C. MS (MH⁺) 224

### 3-(Pyrimidin-4-yl-acetyl)-benzonitrile

A solution of diisopropylamine (4.1ml, 29.3mmol) in dry THF (40ml) is cooled to -60°C and 1.6M BuLi in hexanes (16.6ml, 26.6mmol) is added dropwise. The mixture is then allowed to warm to 20°C. The solution is recooled to -60°C, 4-methylpyrimidine (2.5g, 26.6mmol) is added dropwise, and the solution is stirred at -60°C for 1h. To the resulting solution 3-cyano-N-methoxy-N-methyl-benzamide (5.05g, 26.6mmol) in dry tetrahydrofuran (10ml) is added dropwise. The solution is stirred at -60°C for 1h, then allowed to warm to room temperature. After 18h, water is added to the reaction mixture, and the solvent is removed by evaporation to yield a solid. Water is aded to the mass, and the resulting solid filtered off and washed with water. The product is obtained as a solid, MS (APCI, MH⁺) 224.

The following compounds are prepared in an analogous manner:
3-[(6-Methyl-pyrimidin-4-yl)-acetyl]-benzonitrile, MS (MH⁺) 238.1
3-[(6-Dimethylamino-pyridazin-4-yl)-acetyl]-benzonitrile, MS (MH⁺) 267.3

### Preparation of compounds of formula VIII

### 3-(2-Amino-5-pyrimidin-4-yl-thiazol-4-yl)-benzonitrile

3-(Pyrimidin-4-yl-acetyl)-benzonitrile (1.00g, 4.48mmol) is dissolved in dry dioxane (20ml) and bromine (0.23ml, 4.48mmol) is added dropwise. The mixture is stirred for 30 minutes and then the solvent is evaporated under vacuum at 30°C to give a gum. This residue is dissolved in absolute ethanol (11.5ml). To 7ml of this solution thiourea (0.188g, 2.17mmol) is added and the mixture is heated at a gentle reflux for 6 hours. After cooling the reaction mixture, the solid is filtered off and washed with ethanol. The solid is then suspended in water and aqueous ammonia added dropwise. The resulting solid is filtered off, washed with water and dried. MS (MH⁺) 280.1

The following compounds are prepared in an analogous manner:
3-[2-Amino-5-(6-methyl-pyrimidin-4-yl)-thiazol-4-yl]-benzonitrile, MS (MH⁺) 294.4
3-(2-Amino-5-pyridazin-4-yl-thiazol-4-yl)-benzonitrile, MS (MH⁺) 280.3

### Preparation of Specific Examples

### N-[4-(3-Cyano-phenyl)-5-pyrimidin-4-yl-thiazol-2-yl]-3-methoxy-benzamide (Example 11)

3-(2-Amino-5-pyrimidin-4-yl-thiazol-4-yl)-benzonitrile (90mg, 0.32mmol) is suspended in pyridine (0.75ml) and 3-methoxybenzoyl chloride (165mg, 0.96mmol) is added. A precipitate is formed. The reaction mixture is stirred at room temperature for 18 hours. An excess of water is added to the reaction mixture, and the resulting solid is collected, washed with water and dried. The dried solid is triturated with hot ethanol to give product as a pale powder.m.p.255-256°C, MS (APCI MH⁺) 414. Examples 10 and 13 are prepared analogously.

### 3-[2-(Pyrazin-2-ylamino)-5-pyrimidin-4-yl-thiazol-4-yl]-benzonitrile (Example 12)

3-(pyrimidin-4-yl-acetyl)-benzonitrile (1.00g, 4.48mmol) is dissolved in dry dioxane (11ml) and bromine (0.23ml, 4.48mmol) is added dropwise. The mixture is stirred for 30 minutes and then the solvent is evaporated under vacuum at 30°C to give a solid. This residue is dissolved in absolute ethanol (10.5ml). To 1.5ml of this solution pyrazin-2-yl-thiourea (108mg, 0.70mmol) is then added and the mixture is heated at 70°C for 16 hours. The crude reaction mixture is diluted with water and the resulting solid collected and washed with ethanol. This solid is suspended in water and aqeous ammonia is added to pH9. After 10min. the resulting solid is collected by filtration and washed with ethanol/ethyl acetate mxtures. The solid is dried under vacuum at 70°C to give a powder. m.p.>280°C MS (APCI) MH⁺ and, MH- 358 and 356 respectively. Examples 1 to 9 are prepared analogously.

4-[4-(3-Cyano-phenyl)-5-pyridazin-4-yl-thiazol-2-ylamino]-benzoic acid (Example 15) 3-(pyridazin-4-yl-acetyl)-benzonitrile (1.55g, 6.94mmol) is dissolved in dry dioxane (20ml) at 10-15°C and bromine (0.35ml, 6.9mmol) is added dropwise. The mixture is stirred for 45 minutes and the resulting solid is dissolved in methanol and the solution evaporated to a gum under vacuum at room temperature. This residue is dissolved in dry dimethylformamide (12ml). To 4ml of this solution 4-carboxyphenyl thiourea (0.496g,2.5mmol) is then added and the mixture is heated at 70°C for 20 hours. The reaction mixture is allowed to cool and the solvent removed under vacuum. The residue is triturated with ethyl acetate and then ammonia solution is added. The resulting solid is filtered off, washed with water and dried to give a solid, m.p. >275°C. MS (MH⁺) 400.4. Examples 14 and 16 are prepared analogously.

## Claims

1. A compound of formula 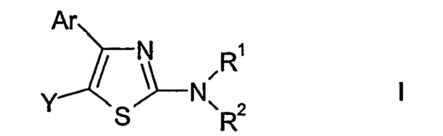 in free or salt form, where
Ar is phenyl substituted by one or more substituents selected from halogen, cyano and C₁-C₈-haloalkyl, or naphthyl,
R¹ is hydrogen, phenyl optionally substituted by one or more substituents selected from halogen, cyano, hydroxy, C₁-C₈-alkyl, C₁-C₈-haloalkyl, C₁-C₈-alkoxy, C₁-C₈-alkoxy-C₁-C₈-alkyl, carboxy, C₁-C₈-alkoxycarbonyl and acyloxy, or R¹ is a 5- or 6- membered monovalent heterocyclic group,
R² is hydrogen, C₁-C₈-alkyl, acyl or -CON(R³)R⁴,
R³ and R⁴ are each independently hydrogen or C₁-C₈-alkyl, or together with the nitrogen atom to which they are attached denote a 5- or 6- membered heterocyclic group, and
Y is a pyrimidinyl or pyridazinyl group, optionally substituted by at least one C₁-C₈-alkyl,
C₁-C₈-alkoxy, C₁-C₈-alkylthio, C₁-C₈-alkylamino, di(C₁-C₈-alkyl)amino or acylamino group.

2. A compound according to claim 1, in which Ar is phenyl optionally substituted by halogen or cyano.

3. A compound according to claim 1 or 2, in which R¹ is phenyl optionally substituted by cyano, carboxy or C₁-C₄-alkoxy, or R¹ is a monovalent 6-membered N-heterocyclic group.

4. A compound according to claim 1, 2 or 3, in which R² is hydrogen, C₁-C₄-alkylcarbonyl, 5-membered heterocyclylcarbonyl, or phenylcarbonyl in which the phenyl moiety is optionally substituted by C₁-C₈-alkoxy.

5. A compound according to one of claims 1 to 4, in which Y is a group of formula 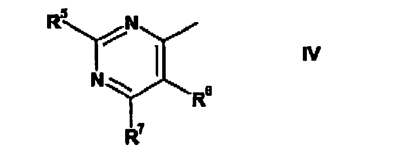 where R⁵ and R⁶ are each hydrogen and R⁷ is hydrogen, C₁-C₄-alkyl or C₁-C₄-alkylthio, or Y is a group of formula 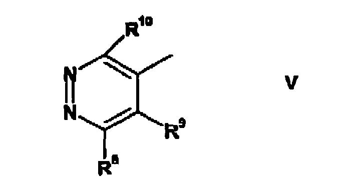 where R⁹ and R¹⁰ are each hydrogen and R⁸ is hydrogen or di(C₁-C₄-alkyl)amino.

6. A compound according to claim 1, in which
Ar is phenyl substituted by halogen or cyano,
R¹ is hydrogen, phenyl optionally substituted by cyano, halogen, carboxy or C₁-C₄-alkoxy-, or R¹ is a monovalent 6-membered N-heterocyclic group,
R² is hydrogen, C₁-C₄-alkylcarbonyl, 5-membered heterocyclylcarbonyl or phenylcarbonyl in which the phenyl moiety is optionally substituted by C₁-C₈-alkoxy, and
Y is pyrimidinyl or pyridazinyl optionally substituteed by C₁-C₄-alkyl, C₁-C₄-alkoxy, C₁-C₄-alkylthio, C₁-C₄-alkylamino, di(C₁-C₄-alkyl) amino or C₁-C₄-alkylcarbonylamino.

7. A compound according to claim 1, in which
Ar is phenyl substituted by cyano meta to the indicated thiazole ring,
R¹ is hydrogen, phenyl substituted by cyano, fluorine, carboxy or C₁-C₄-alkoxy or R¹ is 6-membered N-heterocyclyl having one or two ring nitrogen atoms, optionally substituted by C₁-C₄-alkyl or C₁-C₄-alkoxy,
R² is hydrogen, C₁-C₄-alkylcarbonyl, furylcarbonyl or C₁-C₄-alkoxyphenylcarbonyl, and
Y is a group of formula IV or V as defined in claim 5.

8. A compound according to claim 1, which is selected from :
N-[4-(3-Cyano-phenyl)-5-pyrimidin-4-yl-thiazol-2-yl]-3-methoxy-benzamide;
3-[2-(Pyrazin-2-ylamino)-5-pyrimidin-4-yl-thiazol-4-yl]-benzonitrile; and
4-[4-(3-Cyano-phenyl)-5-pyridazin-4-yl-thiazol-2-ylamino]-benzoic acid

9. A compound according to any one of the preceding claims in combination with an anti-inflammatory, bronchodilatory, antihistamine or anti-tussive drug substance, said compound and said drug substance being in the same or different pharmaceutical composition.

10. A compound according to any one of claims 1 to 9 for use as a pharmaceutical.

11. A pharmaceutical composition comprising a compound according to any one of claims 1 to 9, optionally together with a pharmaceutically acceptable diluent or carrier.

12. The use of a compound according to any one of claims 1 to 9 in the manufacture of a medicament for the treatment of inflammatory or allergic conditions.

13. The use of a compound according to any one of claims 1 to 9 in the manufacture of a medicament for the treatment of an inflammatory or obstructive airways disease.

14. A method of preparing a compound of formula I in free or salt form which comprises
(i)
(A) for the preparation of compounds of formula I where R¹ is optionally substituted phenyl or a 5- or 6- membered heterocyclic group, reacting a compound of formula 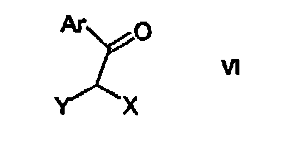 in the form of a salt, where Ar and Y are as defined in claim 1 and X is halogen, with a compound of formula 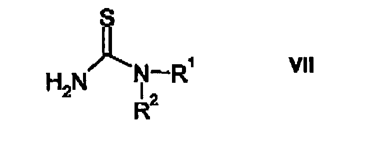 where R¹ is phenyl optionally substituted by one or more substituents selected from halogen, cyano, hydroxy, C₁-C₈-alkyl, C₁-C₈-haloalkyl, C₂-C₈-alkoxy, C₁-C₃-alkoxy-C₁-C₈-alkyl and acyloxy or R¹ is a 5- or 6- membered monovalent heterocyclic group, and R² is H or C₁-C₈-alkyl or
(B) for the preparation of compounds of formula I where R² is acyl or -CON(R³)R⁴, reacting a compound of formula 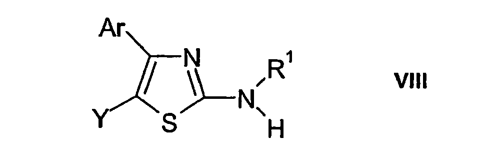 where Ar, R¹ and Y are as hereinbefore defined with, respectively, an acylating derivative of a carboxylic acid or with a compound of formula Cl-CON(R³)R⁴) where R³ and R⁴ are as defined in claim 1, and
(ii) recovering the resultant compound of formula I in free or salt form.

## Patentansprüche

1. Verbindung der Formel 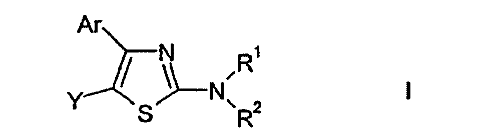 in freier Form oder in Form eines Salzes, worin
Ar für Phenyl, das durch ein oder mehr Substituenten substituiert ist, die ausgewählt sind aus Halogen, Cyano und C₁-C₈-Halogenalkyl, oder für Naphthyl steht,
R¹ für Wasserstoff, Phenyl steht, das optional durch ein oder mehr Substituenten substituiert ist, die ausgewählt sind aus Wasserstoff, Cyano, Hydroxy, C₁-C₈-Alkyl, C₁-C₈-Halogenalkyl, C₁-C₈-Alkoxy, C₁-C₈-Alkoxy-C₁-C₈-alkyl, Carboxy, C₁-C₈-Alkoxycarbonyl und Acyloxy, oder R¹ eine 5- oder 6-gliedrige monovalente heterocyclische Gruppe ist,
R² für Wasserstoff, C₁-C₈-Alkyl, Acyl oder -CON(R³)R⁴ steht,
R³ und R⁴ jeweils unabhängig für Wasserstoff oder C₁-C₈-Alkyl stehen, oder zusammen mit dem Stickstoffatom, an das sie gebunden sind, eine 5- oder 6-gliedrige heterocyclische Gruppe bilden, und
Y für Pyrimidinyl oder Pyridazinyl steht, das optional substituiert ist durch wenigstens ein C₁-C₈-Alkyl, C₁-C₈-Alkoxy, C₁-C₈-Alkylthio, C₁-C₈-Alkylamino, Di-(C₁-C₈-alkyl)-amino oder Acylamino.

2. Verbindung nach Anspruch 1, worin Ar für Phenyl steht, das optional substituiert ist durch Halogen oder Cyano.

3. Verbindung nach Anspruch 1 oder 2, worin R¹ für Phenyl steht, das optional substituiert ist mit Cyano, Carboxy oder C₁-C₄-Alkoxy, oder R¹ für eine monovalente 6-gliedrige N-heterocyclische Gruppe steht.

4. Verbindung nach Anspruch 1, 2 oder 3, worin R² für Wasserstoff, C₁-C₄-Alkylcarbonyl, 5-gliedriges Heterocyclylcarbonyl oder Phenylcarbonyl steht, das im Phenylteil optional substituiert ist durch C₁-C₈-Alkoxy.

5. Verbindung nach einem der Ansprüche 1 bis 4, worin Y für eine Gruppe der folgenden Formel steht 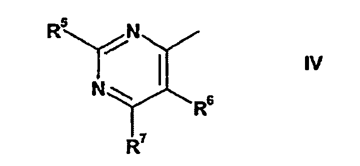 worin R⁵ und R⁶ jeweils für Wasserstoff stehen und R⁷ für Wasserstoff, C₁-C₄-alkyl oder C₁-C₄-Alkylthio steht, oder worin Y für eine Gruppe der folgenden Formel steht 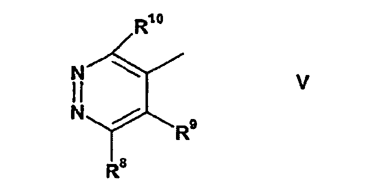 worin R⁹ und R¹⁰ jeweils für Wasserstoff stehen und R⁸ für Wasserstoff oder Di-(C₁-C₄-alkyl)-amino steht.

6. Verbindung nach Anspruch 1, worin
Ar für Phenyl steht, das durch Halogen oder Cyano substituiert ist,
R¹ für Wasserstoff oder Phenyl steht, das optional substituiert ist durch Cyano, Halogen, Carboxy oder C₁-C₄-Alkoxy, oder R¹ für eine monovalente 6-gliedrige N-heterocyclische Gruppe steht,
R² für Wasserstoff, C₁-C₄-Alkylcarbonyl, 5-gliedriges Heterocyclylcarbonyl oder Phenylcarbonyl steht, worin der Phenylteil optional substituiert ist mit C₁-C₈-Alkoxy, und
Y für Pyrimidinyl oder Pyridazinyl steht, welche optional substituiert sind durch C₁-C₄-Alkyl, C₁-C₄-Alkoxy, C₁-C₄-Alkylthio, C₁-C₄-Alkylamino, Di-(C₁-C₄-alkyl)-amino oder C₁-C₄-Alkylcarbonylamino.

7. Verbindung nach Anspruch 1, worin
Ar für Phenyl steht, das in meta-Stellung zum angegebenen Thiazolring durch Cyano substituiert ist,
R¹ für Wasserstoff oder Phenyl steht, das durch Cyano, Fluor, Carboxy oder C₁-C₄-Alkoxy substituiert ist, oder R¹ für ein 6-gliedriges N-Heterocyclyl steht, das ein oder zwei Ringstickstoffatome aufweist, und optional mit C₁-C₄-Alkyl oder C₁-C₄-Alkoxy substituiert ist,
R² für Wasserstoff, C₁-C₄-Alkylcarbonyl, Furylcarbonyl oder C₁-C₄-Alkoxyphenylcarbonyl steht, und
Y für eine Gruppe der Formel IV oder V gemäß Definition von Anspruch 5 steht.

8. Verbindung nach Anspruch 1, die ausgewählt ist aus
N-[4-(3-Cyanophenyl)-5-pyrimidin-4-ylthiazol-2-yl]-3-methoxybenzamid,
3-[2-(Pyrazin-2-ylamino)-5-pyrimidin-4-ylthiazol-4-yl]-benzonitril, und
4-[4-(3-Cyanophenyl)-5-pyridazin-4-ylthiazol-2-ylamino]-benzoesäure.

9. Verbindung nach einem der vorhergehenden Ansprüche in Kombination mit einem antiinflammatorischen, bronchodilatorischen, antihistaminischen oder antitussiven Wirkstoff, wobei diese Verbindung und dieser Wirkstoff in der gleichen oder einer verschiedenen pharmazeutischen Zusammensetzung vorhanden sind.

10. Verbindung nach einem der Ansprüche 1 bis 9 zur Verwendung als ein Pharmazeutikum.

11. Pharmazeutische Zusammensetzung, umfassend eine Verbindung nach einem der Ansprüche 1 bis 9, optional zusammen mit einem pharmazeutisch akzeptablen Verdünnungsmittel oder Träger.

12. Verbindung nach einem der Ansprüche 1 bis 9 zur Herstellung eines Arzneimittels für die Behandlung inflammatorischer oder allergischer Zustände.

13. Verbindung nach einem der Ansprüche 1 bis 9 zur Herstellung eines Arzneimittels für die Behandlung einer inflammatorischen oder obstruktiven Atemwegkrankheit.

14. Verfahren zur Herstellung einer Verbindung der Formel I in freier Form oder in Form eines Salzes, durch
(i)
(A) zur Herstellung von Verbindungen der Formel I, worin R¹ für optional substituiertes Phenyl oder ein 5- oder 6-gliedriges Heterocyclyl steht, Umsetzung einer Verbindung der Formel 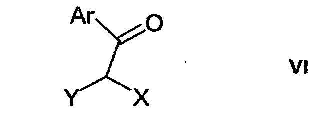 in Form eines Salzes, worin Ar und Y wie im Anspruch 1 definiert sind und X für Halogen steht, mit einer Verbindung der Formel 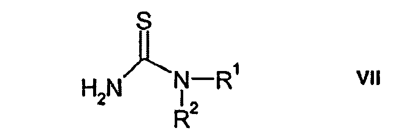 worin R¹ für Phenyl steht, das optional substituiert ist durch ein oder mehr Substituenten, die ausgewählt sind aus Halogen, Cyano, Hydroxy, C₁-C₈-Alkyl, C₁-C₈-Halogenalkyl, C₁-C₈-Alkoxy, C₁-C₈-Alkoxy-C₁-C₈-alkyl und Acyloxy, oder R¹ für eine 5- oder 6-gliedrige monovalente heterocyclische Gruppe steht, und R² für H oder C₁-C₈-Alkyl steht, oder
(B) zur Herstellung von Verbindungen der Formel I, worin R² für Acyl oder -CON(R³)R⁴ steht, Umsetzung einer Verbindung der Formel 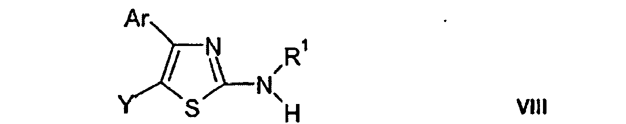 worin Ar, R¹ und Y wie oben definiert sind, mit jeweils einem acylierenden Derivat einer Carbonsäure oder mit einer Verbindung der Formel Cl-CON(R³)R⁴ , worin R³ und R⁴ wie im Anspruch 1 definiert sind, und
(ii) Gewinnung der erhaltenen Verbindung in freier Form oder in Form eines Salzes.

## Revendications

1. Composé de formule : 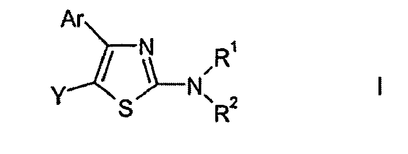 sous forme libre ou sous forme de sel,
formule dans laquelle :
Ar est un groupe phényle substitué par un ou plusieurs substituants choisis entre un halogène, un groupe cyano et haloalkyle en C₁ à C₈, ou naphtyle,
R¹ représente l'hydrogène, un groupe phényle facultativement substitué par un ou plusieurs substituants choisis parmi un halogène, un groupe cyano, hydroxy, alkyle en C₁ à C₈, haloalkyle en C₁ à C₈, alkoxy en C₁ à C₈, alkoxy en C₁ à C₈-alkyle en C₁ à C₈, carboxy, alkoxy en C₁ à C₈-carbonyle et acyloxy, ou bien R¹ est un groupe hétérocyclique monovalent à 5 ou 6 chaînons,
R² représente l'hydrogène, un groupe alkyle en C₁ à C₈, acyle ou -CON(R³)R⁴,
R³ et R⁴ représentent chacun indépendamment l'hydrogène ou un groupe alkyle en C₁ à C₈ ou, conjointement avec l'atome d'azote auquel ils sont fixés, représentent un groupe hétérocyclique à 5 ou 6 chaînons, et
Y est un groupe pyrimidinyle ou pyridazinyle, facultativement substitué par au moins un groupe alkyle en C₁ à C₈, alkoxy en C₁ à C₈, alkylthio en C₁ à C₈, alkylamino en C₁ à C₈, di-(alkyle en C₁ à C₈)-amino ou acylamino.

2. Composé selon la revendication 1, dans lequel Ar est un groupe phényle facultativement substitué par un halogène ou un groupe cyano.

3. Composé selon la revendication 1 ou 2, dans lequel R¹ est un groupe phényle facultativement substitué par un groupe cyano, carboxy ou alkoxy en C₁ à C₄, ou bien R¹ est un groupe N-hétérocyclique monovalent à 6 chaînons.

4. Composé selon la revendication 1, 2 ou 3, dans lequel R² représente l'hydrogène, un groupe alkyle en C₁ à C₄-carbonyle, un groupe hétérocyclylcarbonyle à 5 chaînons, ou phénylcarbonyle dans lequel le groupement phényle est facultativement substitué par un groupe alkoxy en C₁ à C₈.

5. Composé selon l'une quelconque des revendications 1 à 4, dans lequel Y est un groupe de formule : 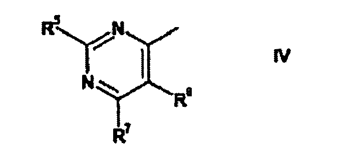 dans laquelle R⁵ et R⁶ représentent chacun l'hydrogène, et R⁷ représente l'hydrogène, un groupe alkyle en C₁ à C₄ ou alkylthio en C₁ à C₄, ou bien Y est un groupe de formule : 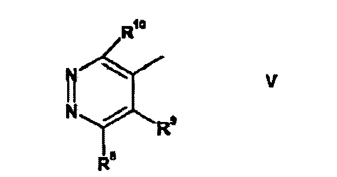 dans laquelle R⁹ et R¹⁰ représentent chacun l'hydrogène, et R⁸ représente l'hydrogène ou un groupe di-(alkyle en C₁ à C₄)-amino.

6. Composé selon la revendication 1, dans lequel :
Ar est un groupe phényle substitué par un halogène ou un groupe cyano,
R¹ représente l'hydrogène, un groupe phényle facultativement substitué par un groupe cyano, un halogène, un groupe carboxy ou alkoxy en C₁ à C₄,
ou bien R¹ est un groupe N-hétérocyclique monovalent à 6 chaînons,
R² représente l'hydrogène, un groupe alkyle en C₁ à C₄-carbonyle, hétérocyclylcarbonyle à 5 chaînons ou phénylcarbonyle, dans lequel le groupement phényle est facultativement substitué par un groupe alkoxy en C₁ à C₈, et
Y est un groupe pyrimidinyle ou pyridazinyle facultativement substitué par un groupe alkyle en C₁ à C₄, alkoxy en C₁ à C₄, alkylthio en C₁ à C₄, alkylamino en C₁ à C₄, di-(alkyle en C₁ à C₄)-amino, ou alkyle en C₁ à C₄-carbonylamino.

7. Composé selon la revendication 1, dans lequel :
Ar est un groupe phényle substitué par un groupe cyano en méta par rapport au cycle thiazole indiqué,
R¹ représente l'hydrogène, un groupe phényle substitué par un groupe cyano, le fluor, un groupe carboxy ou alkoxy en C₁ à C₄, ou bien R¹ est un groupe N-hétérocyclyle à 6 chaînons ayant un ou deux atomes d'azote dans le cycle, facultativement substitué par un groupe alkyle en C₁ à C₄ ou alkoxy en C₁ à C₄,
R² représente l'hydrogène, un groupe alkyle en C₁ à C₄-carbonyle, furylcarbonyle ou alkoxy en C₁ à C₄-phénylcarbonyle, et
Y est un groupe de formule IV ou V tel que défini dans la revendication 5.

8. Composé selon la revendication 1, qui est choisi entre les composés suivants :
le N-[4-(3-cyano-phényl)-5-pyrimidin-4-yl-thiazol-2-yl]-3-méthoxybenzamide ;
le 3-[2-(pyrazin-2-ylamino)-5-pyrimidin-4-yl-thiazol-4-yl]-benzonitrile ; et
l'acide 4-[4-(3-cyano-phényl)-5-pyridazin-4-yl-thiazol-2-ylamino]-benzoïque.

9. Composé selon l'une quelconque des revendications précédentes, en combinaison avec une substance médicamenteuse anti-inflammatoire, bronchodilatatrice, anti-histaminique ou anti-tussive, ledit composé et ladite substance médicamenteuse se trouvant dans la même composition pharmaceutique ou dans une composition pharmaceutique différente.

10. Composé selon l'une quelconque des revendications 1 à 9, à utiliser comme médicament.

11. Composition pharmaceutique comprenant un composé selon l'une quelconque des revendications 1 à 9, éventuellement conjointement avec un diluant ou support pharmaceutiquement acceptable.

12. Utilisation d'un composé selon l'une quelconque des revendications 1 à 9 dans la fabrication d'un médicament pour le traitement d'états inflammatoires ou allergiques.

13. Utilisation d'un composé selon l'une quelconque des revendications 1 à 9 dans la fabrication d'un médicament pour le traitement d'une maladie inflammatoire ou obstructrice des voies aériennes.

14. Procédé de préparation d'un composé de formule I, sous forme libre ou sous forme d'un sel, qui comprend :
(i)
(A) pour la préparation de composés de formule I dans lesquels R¹ est un groupe phényle facultativement substitué ou un groupe hétérocyclique à 5 ou 6 chaînons, la réaction d'un composé de formule : 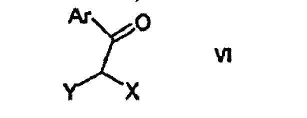 sous forme d'un sel, où Ar et Y sont tels que définis dans la revendication 1 et X représente un halogène, avec un composé de formule : 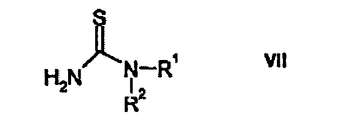 dans laquelle R¹ est un groupe phényle facultativement substitué par un ou plusieurs substituants choisis entre un halogène, un groupe cyano, hydroxy, alkyle en C₁ à C₈, haloalkyle en C₁ à C₈, alkoxy en C₁ à C₈, alkoxy en C₁ à C₈-alkyle en C₁ à C₈ et acyloxy, ou bien R¹ est un groupe hétérocyclique monovalent à 5 ou 6 chaînons, et R² représente H ou un groupe alkyle en C₁ à C₈, ou
(B) pour la préparation de composés de formule I dans lesquels R² est un groupe acyle ou -CON(R³)R⁴, la réaction d'un composé de formule : 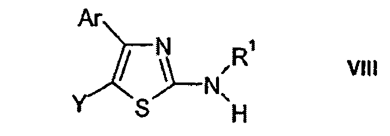 dans laquelle Ar, R¹ et Y sont tels que définis ci-dessus, avec, respectivement, un dérivé acylant d'un acide carboxylique ou avec un composé de formule Cl-CON(R³)R⁴, dans laquelle R³ et R⁴ sont tels que définis dans la revendication 1, et
(ii) la récupération du composé résultant de formule I sous forme libre
ou sous forme d'un sel.
